(19) Europäisches Patentamt
European Patent Office
Office européen des brevets

(11) Publication number: **0 211 573 B1**

(12) # EUROPEAN PATENT SPECIFICATION

(45) Date of publication of the patent specification: **25.01.89**

(51) Int. Cl.⁴: **C07D 317/70,** A61K 31/335, A61K 31/36

(21) Application number: **86305704.8**

(22) Date of filing: **24.07.86**

(54) **Novel antimicrobial active agent and its production.**

(30) Priority: **24.07.85 JP 161791/85**

(43) Date of publication of application: **25.02.87 Bulletin 87/9**

(45) Publication of the grant of the patent: **25.01.89 Bulletin 89/4**

(84) Designated Contracting States: **DE FR GB**

(56) References cited: **CH-A- 636 870**

(73) Proprietor: **NICHIREI CORPORATION, 3-3-23 Misaki-cho Chiyoda-ku, Tokyo(JP)**

(72) Inventor: **Masuda, Kazuaki, 3-24-5 Megurita-cho, Higashimurayama-shi Tokyo(JP)**

(74) Representative: **Woods, Geoffrey Corlett, J.A. KEMP & CO. 14 South Square Gray's Inn, London WC1R 5EU(GB)**

EP 0 211 573 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to compounds having antimicrobial activity, to their preparation and to pharmaceutical compositions containing them.

Antimicrobial agents, for example synthetic sulfa-drugs and antibiotics such as penicillin have been well known and applied in practice. Recently, many kinds of substances against antibiotic-resistant microbial strains have been synthesized and further more effective substances against resistant strains are required.

We have now found that a substance extracted from Tritonia crocosmaeflora Lemoin (Iridaceae) has antimicrobial activity against microorganisms such as Bacillus subtilis, Staphylococcus aureus, Sarcina lutea, Escherichia coli and Saccharomyces sake. We have also found that the substance corresponds to 5,8-dihydroxy-2,3-dimethoxy-6,7-methylenedioxy-1,4-naphthoquinone and its structural isomers.

Accordingly, this invention provides compounds of the formula:

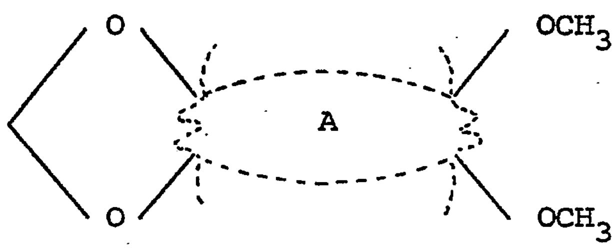

(I)

wherein ring system A is

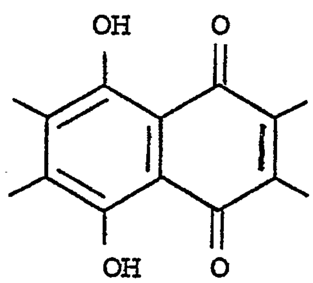

,

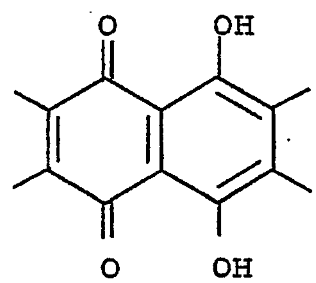

or

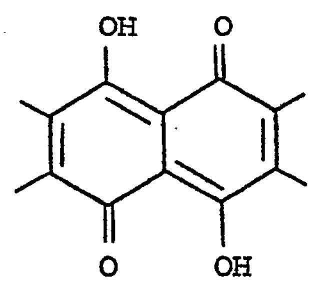

.

More particularly, the present invention provides compounds 5,8-dihydroxy-2,3-dimethoxy-6,7-methylenedioxy-1,4-naphthoquinone, 1,4-dihydroxy-2,3-dimethoxy-6,7-methylenedioxy-5,8-naphthoquinone and 1,5-dihydroxy-2,3-dimethoxy-6,7-methylenedioxy-4,8-naphthalenedione.

The invention further provides said substances having the following physico-chemical properties.

| | | | |
|---|---|---|---|
| a) | molecular weight: | | 294 |
| b) | melting point: | | 185–189 187 °C |
| c) | specific rotation: | | $[\alpha]_D^{20} = 0°$ |
| d) | ultraviolet absorption spectrum: | | as shown in Figure 1 of the accompanying drawings |
| e) | infrared absorption spectrum: | | as shown in Figure 2 of the accompanying drawings |
| f) | solubility: | | easily soluble under neutral and acidic conditions in organic solvents such as methanol, acetone, ethyl acetate, chloroform or benzene; easily soluble under alkaline conditions in water |
| g) | color reaction: | negative:<br>positive: | anthrone-$H_2SO_4$, ninhidrin and Dragendorf reactions;<br>ferrous chloride reaction |
| h) | nature: | | acidic substance (water soluble under alkaline conditions) |
| i) | color and crystalline form: | | deep red-brown fine needle crystals |

These properties correspond to 5,8-dihydroxy-2,3-dimethoxy-6,7-methylenedioxy-1,4-naphthoquinone and its two tautomers 1,4-dihydroxy-2,3-dimethoxy-6,7-methylenedioxy-5,8-naphthoquinone and 1,5-dihydroxy-2,3-dimethoxy-6,7-methylenedioxy-4,8-naphthalenedione. The two tautomers are referred to herein as the structural isomers.

The present invention also includes a process for the production of 5,8-dihydroxy-2,3-dimethoxy-6,7-methylenedioxy-1,4-naphthoquinone and its structural isomers, which process comprises

(i) extracting bulb tissue of Tritonia crocosmaeflora Lemoin (Iridaceae) with an organic solvent, and
(ii) obtaining the desired substance from the extract thus obtained.

Step (ii) may be achieved by subjecting the extract to liquid-liquid distribution extraction with water and an organic solvent, or to solid-liquid extraction with an organic solvent after drying up the extract, and then treating the thus obtained organic extract by absorption chromatography and gel-filtration chromatography repeatedly if necessary.

The invention additionally provides a pharmaceutical composition comprising a pharmaceutically acceptable carrier or diluent and, as active ingredient, a compound or substance of the invention.

A raw material for use in the present invention, Tritonia crocosmaeflora Lemoin (Iridaceae) is a hybrid of Crocosmia aurea Pappe (Iridaceae) and Tritonia pottsii Brown, N E (Iridaceae), and is known for the flowers of several kinds of colours from yellow to orange-red. In order to obtain 5,8-dihydroxy-2,3-dimethoxy-6,7-methylenedioxy-1,4-naphthoquinone and its structural isomers, whole or part of bulbs and rhizomes of Tritonia crocosmaeflora Lemoin (Iridaceae) are used as a raw material. These can be used as a whole body, but are preferably cut up. Also these raw materials in dried form can be used.

Cut up bulbs, rhizomes and roots are extracted with an organic solvent. Examples of organic solvents are methanol, ethanol, acetone, ethyl acetate, butyl acetate, benzene, toluene, xylene, diethyl ether, dichloromethane, chloroform, carbon tetrachloride, hexane and mixtures of two or more thereof. Extraction can be performed at ambient temperature, and can also be done under reflux. The extraction time can be selected according to the size and quality of the material being treated. It is usually from 1 to 72 hours. The extracting solvent can also be changed and used repeatedly.

The thus obtained extract is suitably separated by filtration or centrifugation or a combination thereof. The separated extract which contains impurities is typically subjected to absorption chromatography and gel-filtration in order to purify the active principle. Before these treatments, the extract is preferably treated by liquid-liquid distribution extraction with a mixture of water and organic solvent or is dried up by means of a suitable procedure and the solid material thus obtained is treated by extraction with an organic solvent. Examples of organic solvents are the same as previously mentioned. The thus obtained extract can be dried up or concentrated in vacuo or under normal pressure, extracted with a mixture of water and one or more organic solvents such as mentioned hereinabove and the organic layer separated therefrom by phase separation or centrifugation.

Since the substance of the present invention is insoluble in organic solvents and soluble in water under alkaline conditions and insoluble in water and soluble in organic solvent under acidic conditions, the extract obtained by the operation hereinabove can be purified by repeated phase transfer between organic solvent and water under alkaline and acidic conditions. For example, aqueous ammonia, sodium carbonate, sodium bicarbonate or sodium hydroxide can be added to a mixture of organic solvent and water. The substance then is transferred to the aqueous phase which is separated by centrifugation. The separated aqueous phase is acidified by adding an acid such as acetic acid or hydrochloric acid and mixed with an organic solvent to transfer the substance thereinto. These operations are repeated to remove impurities.

The extract is treated by absorption chromatography such as silica-gel column chromatography or thinlayer chromatography for further purification. Examples of developing solvents are benzene, chloroform, dichloromethane, ethyl acetate, butyl acetate, carbon tetrachloride, methanol and ethanol, or a mixture thereof. Acetio acid, hydrochloric acid or the like can be added if required. Gel-filtration using a cross-linked dextran such as Sephadex LH-20 (Pharmacia Co) can also be used for further purification.

Physico-chemical properties of the novel compound [Ia] of the present invention are illustrated as follows.

a) molecular weight: 294
b) melting point: 185–189 (187)°C
c) specific rotation: $[\alpha]^{20}_{D} = 0°$
d) ultraviolet absorption spectrum: shown in Fig. 1
e) infrared absorption spectrum: shown in Fig. 2
f) solubility: easily soluble at neutral and acidic condition in organic solvent such as methanol, acetone, ethyl acetate, chloroform or benzene;
easily soluble at alkaline condition in water
g) color reaction: negative: anthrone-$H_2SO_4$, ninhidrin, Dragendorf reaction;
positive: ferrous chloride reacton
h) nature: acidic substance (water soluble in alkaline condition)
i) color and crystalline form: deep red-brown fine needle crystals.

Chemical structure of the novel compound is determined as 5,8-dihydroxy-2,3-dimethoxy-6,7-methylenedioxy-1,4-naphthoquinone and its structural isomers, which is shown as follows.

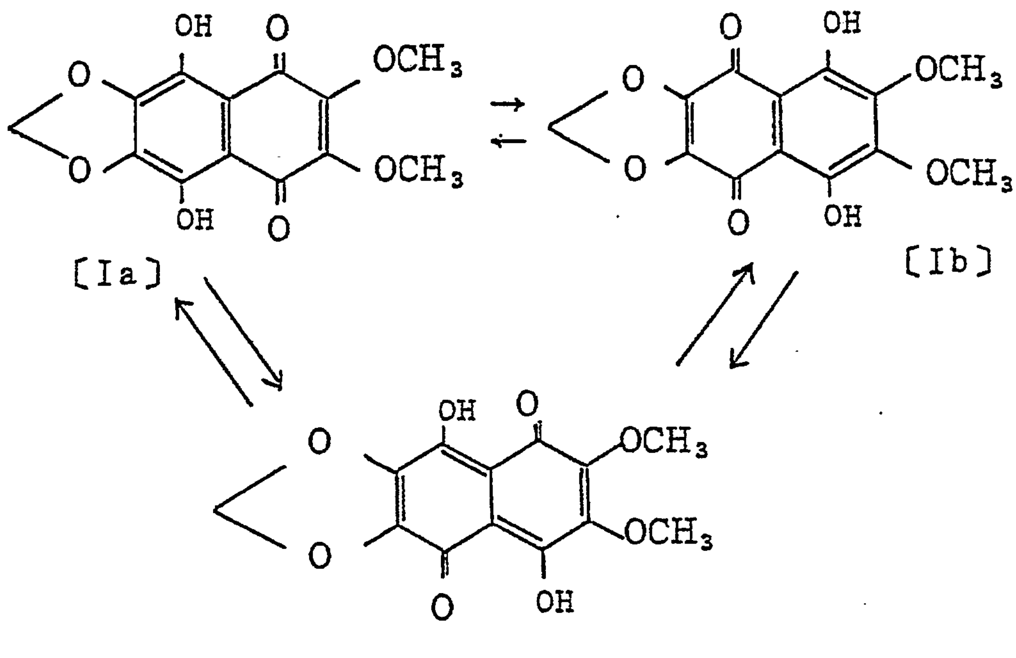

[Ia] [Ib]

[Ic]

These isomers are easily tautomerized in a solution. Therefore these tautomers are included in the present invention.

Biological properties of the compound of the present invention were investigated as follows:

(1) Antimicrobial activity:

1) Preparation:
The compound (Ia) dissolved in chloroform is immersed in paper disc, diameter 6 mm, and dried on a filter paper.

2) Antimicrobial activity asasy:

Microbial strains shown in Table 1 are inoculated in agar medium, and paper discs hereinabove 1) are placed thereon then incubated at the temperature shown in the Table. Diameter of inhibitory zone is measured after 12 hours incubation. Results are shown in Table 1.

Table 1

| Microbial strains Samples density (μg/ml) | Bacillus subtilis | Staphylococcus aureus | Sarcina lutea | Escherichia coli | Saccharomyces sake |
|---|---|---|---|---|---|
| 448 | 15.50* | 8.45 | 7.4 | + | 7.3 |
| 224 | 13.30* | 7.70 | 7.4 | + | 7.0 |
| 112 | 13.30 | +* | 7.4 | + | + |
| 56 | 8.60 | + | + | ± | + |
| 28 | 9.50 | + | ± | – | – |
| 14 | 7.10 | – | ± | – | – |
| 7 | ±** | – | – | – | – |
| Culture Temp. (°C) | 37 | 37 | 37 | 37 | 27 |

*: mm

** ±: inhibitory zone slightly observed

***+: inhibitory zone < 7 mm

As illustrated a compound of the present invention has antimicrobial activity.

(2) Acute toxicity:

A compound, 1–5 mg, dissolved in physiological saline 0.2 ml containing gum arabic is administered intra peritoneally in mice, body weight 25 g, 10 mice in one group. $LD_{50}$ is over 200 mg/kg.

The following Example illustrates the present invention but is not construed as limiting.

<u>Example</u>

A mixture of dried bulb, rhizome and root of <u>Tritonia crocosmaeflora</u> Lemoin (Iridaceae) 4.5 kg was cutted and methanol was added thereto to extract for 72 hours. Filtered solid part was twice repeatedly extracted by the same way and the combined extract was concentrated in cacuo up to 1 lit. The concentrate was extracted three times with benzene which was concentrated to syrup.

The syrup dissolved in chloroform 10 ml containing 1% acetic acid was chromatographed with column of Kiesel-gel 60 (Merck) 50 g, diameter 70 mm × height 170 mm, eluted with chloroform containing 1% acetic acid.

Red colored band, next to the yellow band of the first, was collected to obtain the eluate approximately 600 ml, which was washed with equal amount of water to remove off acetic acid. Chloroform layer was dried up in vacuo. Solid material was dissolved in methanol 5 ml, allowed to stand overnight, removed off precipitated colorless crystals and the filtrate was concentrated in vacuo up to approximately 2 ml. The concentrate was chromatographed with Sephadex LH-20 (Pharmacia Co.) 200 g, column diameter 25 mm × height 450 mm, developer methanol. The first yellow band and the second red band were discarded and the third red band was collected to obtain the eluate 70 ml. The eluate was allowed to stand over-night at –20°C and precipitated deep red-brown fine needle crystals were collected by filtration to obtain the product 3.4 mg.

## Claims

1. A compound of the formula (I)

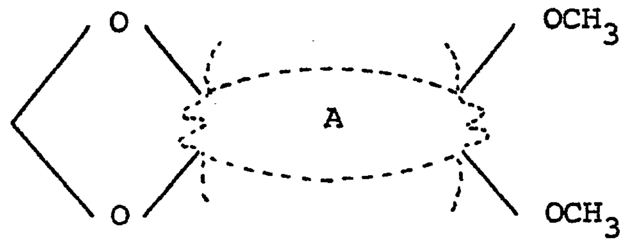

(I)

wherein ring system A is

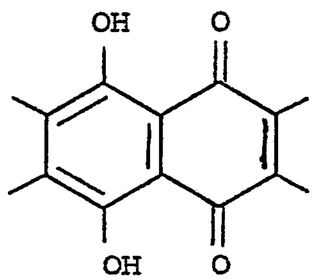

,

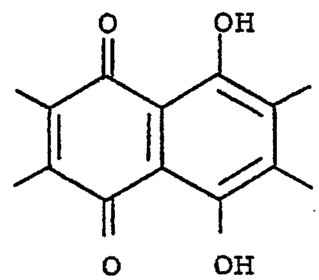

.

or

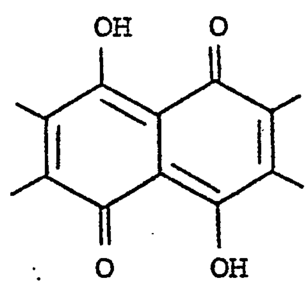

.

2. A compound according to claim 1, which is 5,8-dihydroxy-2,3-dimethoxy-6,7-methylenedioxy-1,4-naphthoquinone.

3. A compound according to claim 1, which is 1,4-dihydroxy-2,3-dimethoxy-6,7-methylenedioxy-5,8-naphthoquinone.

4. A compound according to claim 1, which is 1,5-dihydroxy-2,3-dimethoxy-6,7-methylenedioxy-4,8-naphthalene-dione.

5. A compound according to claim 1 which has the following physico-chemical properties:

| | | | |
|---|---|---|---|
| a) | molecular weight: | | 294 |
| b) | melting point: | | 185–189 187 °C |
| c) | specific rotation: | | $[\alpha]_D^{20} = 0°$ |
| d) | ultraviolet absorption spectrum: | | as shown in Figure 1 of the accompanying drawings |
| e) | infrared absorption spectrum: | | as shown in Figure 2 of the accompanying drawings |
| f) | solubility: | | easily soluble under neutral and acidic conditions in organic solvents such as methanol, acetone, ethyl acetate, chloroform or benzene; easily soluble under alkaline conditions in water |
| g) | color reaction: | negative:<br>positive: | anthrone-$H_2SO_4$, ninhidrin and Dragendorf reactions;<br>ferrous chloride reaction |
| h) | nature: | | acidic substance (water soluble under alkaline conditions) |
| i) | color and crystalline form: | | deep red-brown fine needle crystals |

6. A process for production of a compound as claimed in claim 5, which process comprises
(i) extracting bulb tissue of <u>Tritonia crocosmaeflora</u> Lemoin (Iridaceae) with an organic solvent, and

(ii) obtaining the desired substance from the extract thus obtained.

7. A process according to claim 6 wherein the bulb tissue is bulb material which has been cut up.

8. A process according to claim 6 or 7 wherein the organic solvent for extraction is methanol, ethanol, acetone, ethyl acetate, butyl acetate, benzene, toluene, xylene, diethyl ether, dichloromethane, chloroform, carbon tetrachloride, hexane or a mixture of two or more thereof.

9. A process according to any one of claims 6 to 8 wherein the extract is subjected to liquid-liquid or solid-liquid organic solvent distribution extraction, absorption chromatography and gel-filtration.

10. A process according to claim 9 wherein the absorption chromatography is performed using silica-gel or one of its relative carriers and the gel-filtration is performed using a cross-linked dextran or one of its relative carriers.

11. A pharmaceutical composition comprising a pharmaceutically acceptable carrier or diluent and, as active ingredient, a compound as claimed in any one of claims 1 to 5 or which has been produced by a process as claimed in any one of claims 6 to 10.

**Patentansprüche**

1. Verbindung der Formel (I)

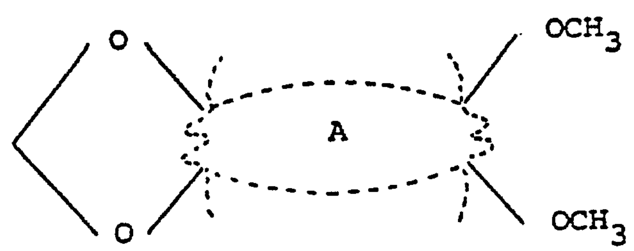

(I)

worin das Ringsystem A

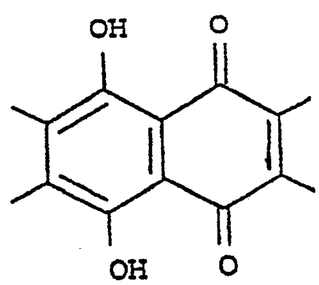

,

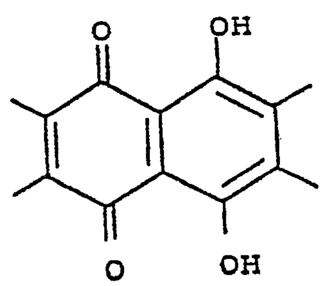

oder

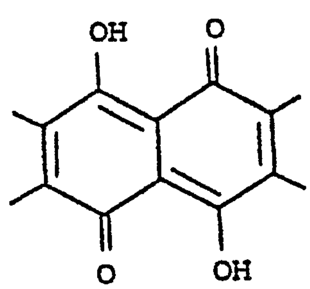

bedeutet.

2. Verbindung nach Anspruch 1, welche 5,8-Dihydroxy-2,3-dimethoxy-6,7-methylendioxy-1,4-naphthochinon darstellt.

3. Verbindung nach Anspruch 1, welche 1,4-Dihydroxy-2,3-dimethoxy-6,7-methylendioxy-5,8-naphthochinon darstellt.

4. Verbindung nach Anspruch 1, welche 1,5-Dihydroxy-2,3-dimethoxy-6,7-methylendioxy-4,8-naphthalen-dion darstellt.

5. Verbindung nach Anspruch 1, welche die folgenden physikalisch-chemischen Eigenschaften aufweist:

| | | | |
|---|---|---|---|
| a) | Molekulargewicht: | | 294 |
| b) | Schmelzpunkt: | | 185–189 187°C |
| c) | spezifische Drehung: | | $[\alpha]_D^{20} = 0°$ |
| d) | Ultraviolett-Apsorptionsspektrum: | | wie in Figur 1 der beiliegenden Zeichnung |
| e) | Iinfrarot-Absorptionsspektrum: | | wie in der Figur 2 der beiliegenden Zeichnung |
| f) | Löslichkeit: | | leicht löslich unter neutralen und sauren Bedingungen in organischen Lösungsmitteln wie Methanol, Azeton, Ethylazetat, Chloroform oder Benzol; leicht löslich unter alkalischen Bedingungen in Wasser |
| g) | Farbreaktion: | negativ:<br>positiv: | Anthron-$H_2SO_4$; Ninhydrin- und Dragendorf-Reaktionen;<br>Ferrochlorid-Reaktion |
| h) | Charakter: | | saure Substanz (wasserlöslich unter alkalischen Bedingungen) |
| i) | Farbe und Kristallform: | | tiefrot-braune feine Nadelkristalle |

6. Verfahren zur Herstellung einer Verbindung nach Anspruch 5 durch
(i) Extraktion von Zwiebel- bzw. Knollengewebe von Tritonia crocosmaeflora Lemoin (Iridaceen) mit einem organischen Lösungsmittel, und
(ii) Gewinnung der gewünschten Substanz aus dem dabei erhaltenen Extrakt.

7. Verfahren nach Anspruch 6, wobei das Zwiebel- bzw. Knollengewebe aufgeschnittenes Zwiebel- bzw. Knollenmaterial darstellt.

8. Verfahren nach Anspruch 6 oder 7, wobei als organisches Lösungsmittel Methanol, Ethanol, Azeton, Ethylazetat, Butylazetat, Benzol, Toluol, Xylol, Diethyläther, Dichlormethan, Chloroform, Kohlenstofftetrachlorid, Hexan, oder eine Mischung von zwei oder mehreren von diesen verwendet wird.

9. Verfahren nach einem der vorhergehenden Ansprüche 6 bis 8, wobei das Extrakt einer Flüssig-Flüssig- oder Fest-Flüssig-Verteilungsextraktion mit einem organischen Lösungsmittel, einer Absorptionschromatographie und einer Gelfiltration unterworfen wird.

10. Verfahren nach Anspruch 9, wobei die Absorptionschromatographie unter Verwendung von Silicagel oder eines diesem entsprechenden Trägermaterials und die Gelfiltration unter Verwendung eines vernetzten Dextrans oder eines diesem entsprechenden Trägermaterials durchgeführt wird.

11. Pharmazeutische Zusammensetzung enthaltend einen pharmazeutisch verträglichen Träger oder Verdünnungsmittel und, als Wirkstoff, eine Verbindung gemäß einem der Ansprüche 1 bis 5 oder hergestellt nach einem Verfahren nach einem der Ansprüche 6 bis 10.

## Revendications

1. Composé de la formule (I)

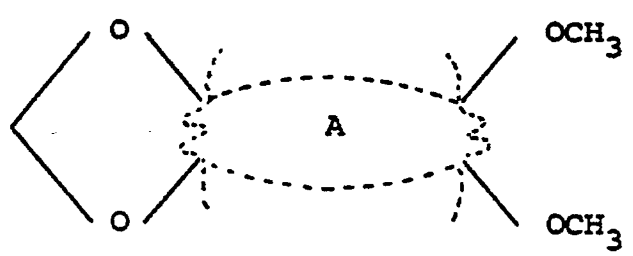

(I)

dans laquelle le système cyclique A est

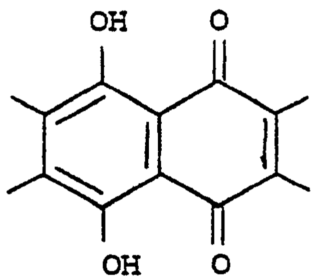 , 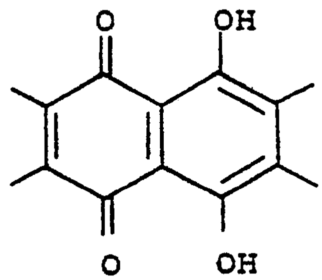

ou

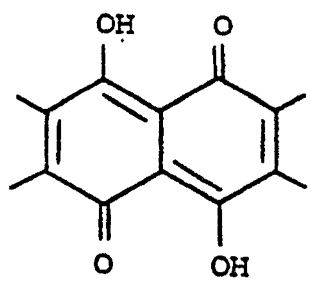 .

2. Composé selon la revendication 1 qui est 5,8-dihydroxy-2,3-diméthoxy-6,7-méthylènedioxy-1,4-naphtoquinone.

3. Composé selon la revendication 1 qui est 1,4-dihydroxy-2,3-diméthoxy-6,7-méthylènedioxy-5,8-naphtoquinone.

4. Composé selon la revendication 1 qui est 1,5-dihydroxy-2,3-diméthoxy-6,7-méthylènedioxy-4,8-naphtalène-dione.

5. Composé selon la revendication 1 qui a les propriétées physico-chimiques suivantes:

| | | | |
|---|---|---|---|
| a) | poids moléculaire: | | 294 |
| b) | poids de fusion: | | 185–189 187 °C |
| c) | rotation spécifique: | | $[\alpha]_D^{20} = 0°$ |
| d) | spectre d'absorption ultraviolets: | | comme représenté sur la figure 1 du dessin joint |
| e) | spectre d'absorption des infra-rouges: | | comme représenté sur la figure 2 du dessin joint |
| f) | solubilité: | | facilement soluble dans des conditions neutres et acides dans des solvants organiques tels que méthanol, acétone, acétate d'éthyle, chloroforme ou benzène; facilement soluble dans l'eau dans des conditions alcalines |
| g) | réaction coloreé: | négative: | réaction à l'anthrone $H_2SO_4$, à la ninhydrine et au réactif de Dragendorf |
| | | positive: | réaction au chlorure ferreux |
| h) | nature: | | substance acide (soluble dans l'eau dans des conditions) |
| i) | couleur et forme cristalline: | | fins cristaux aciculaires brun rouge foncé |

6. Procédé pour la préparation d'un composé selon la revendication 5, lequel procédé consiste à:
(i) soumettre à extraction le tissu bulbaire de Tritonia crocosmaeflore Lemoin (Iridacée) avec un solvant organique et,
(ii) obtenir la substance désirée de l'extrait ainsi obtenu.

7. Procédé selon la revendication 6, dans lequel le tissu bulbaire est constitué par des bulbes qui ont été coupés.

8. Procédé selon la revendication 6 ou 7, dans lequel le solvant organique pour l'extraction est l'un des suivants: méthanol, éthanol, acétone, acétate d'éthyle, acétate de butyle, benzène, toluène, xylène, diéthyl éther, dichlorométhane, chloroforme, tétrachlorure de carbone, hexane ou un mélange de deux ou plusieurs de ces solvants.

9. Procédé selon l'une quelconque des revendications 6 à 8, dans lequel l'extrait est soumis à une extraction par distribution liquide-liquide ou solide-solvant organique liquide, chromatographie par absorption et filtration sur gel.

10. Procédé selon la revendication 9, dans lequel la chromatographie par absorption est effectuée en utilisant du gel de silice ou l'un de ses véhicules dérivés et la filtration sur gel est effectuée en utilisant un dextrane réticulé ou l'un de ses véhicules dérivés.

11. Composition pharmaceutique contenant un véhicule ou un diluant pharmaceutiquement acceptable et, comme ingrédient actif, un composé selon l'une quelconque des revendications 1 à 5, ou qui a été préparé par un procédé selon l'une quelconque des revendications 6 à 10.

FIG. 1

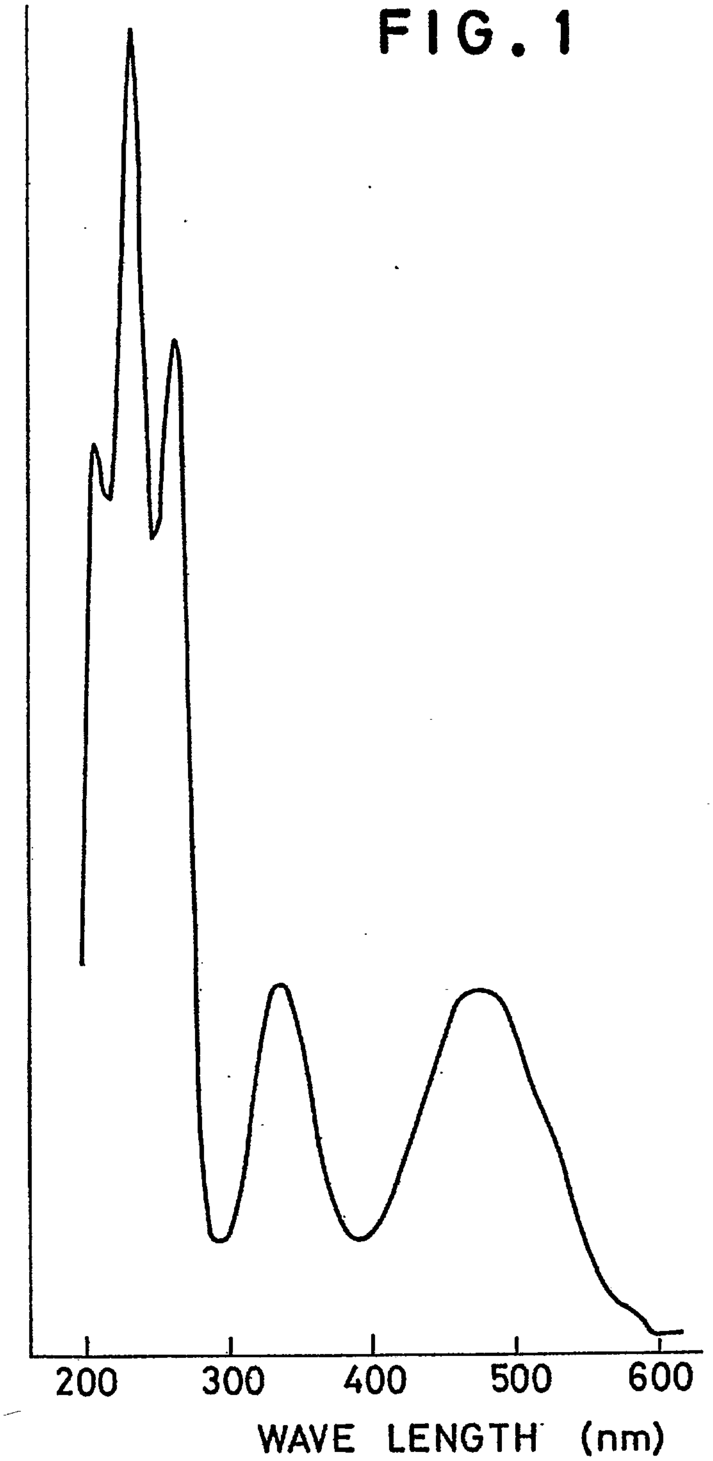

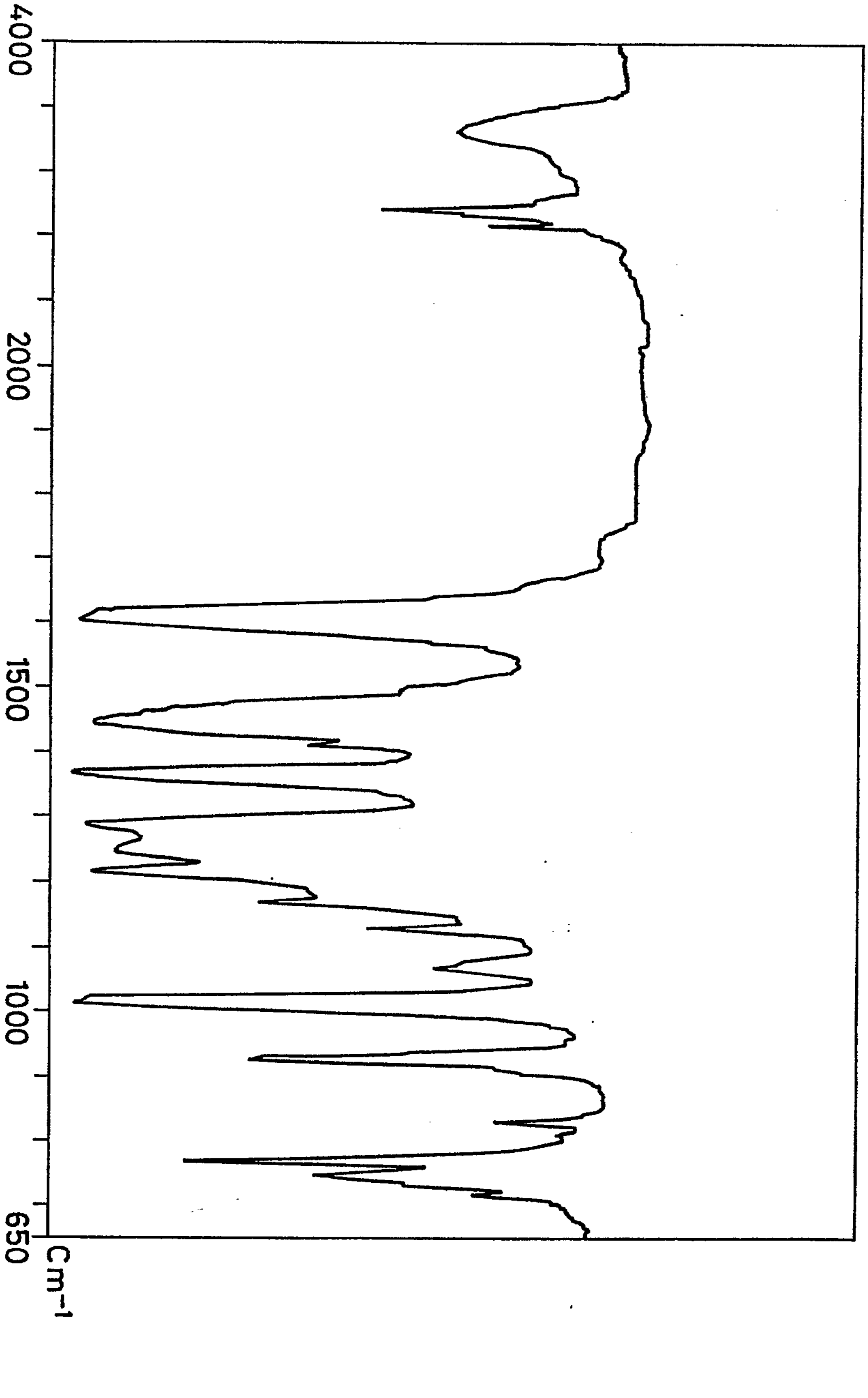


FIG. 2